# EUROPÄISCHE PATENTSCHRIFT

(11) **EP 1 858 560 B1**
(45) Veröffentlichungstag und Bekanntmachung des Hinweises auf die Patenterteilung: **25.12.2013**
(21) Anmeldenummer: 06707513.5
(22) Anmeldetag: 10.03.2006
(51) Int. Cl.: A61L 2/20, A61L 2/26, B65B 55/10, B67C 7/00

(54) **VERFAHREN UND VORRICHTUNG BETREFFEND DAS STERILABFÜLLEN VON FLÜSSIGKEITEN**
METHOD AND DEVICE RELATING TO THE STERILE FILLING OF LIQUIDS
PROCEDE ET DISPOSITIF CONCERNANT LE REMPLISSAGE STERILE DE LIQUIDES

(30) Priorität: 16.03.2005 DE 102005012507
(43) Veröffentlichungstag der Anmeldung: 28.11.2007
(73) Patentinhaber: KRONES Aktiengesellschaft, 93073 Neutraubling (DE)
(72) Erfinder: FISCHER, Sven, 93083 Obertraubling (DE)
(86) Internationale Anmeldenummer: PCT/EP2006/002209
(87) Internationale Veröffentlichungsnummer: WO 2006/097243

(56) Entgegenhaltungen:
- WO-A-02/064174
- DE-A1- 4 305 478
- US-A- 6 024 917
- US-A- 6 119 433
- US-A- 6 120 730
- US-B1- 6 692 684

## Beschreibung

Die Erfindung betrifft eine Vorrichtung und ein Verfahren, die das Sterilabfüllen von Flüssigkeiten betreffen.

Die sterile Abfüllung von beispielsweise Getränken ist für eine lange Mindesthaltbarkeitsdauer wichtig. Hierzu ist es bekannt, Flaschen vor der Befüllung zu sterilisieren und mit sterilen Getränken zu füllen und die Flaschen anschließend unter Wahrung der Sterilität zu verschließen.

Aus der DE 37 01 079 A1 ist beispielsweise ein Verfahren zum Entkeimen von Verpackungsbehältern bekannt. Hier wird auf die vorgewärmten Behälter Wasserstoffperoxid aufgeblasen, sodass sich an den Wänden des Behälters ein Kondensatfilm bildet. Anschließend wird das Entkeimungsmittel durch Spülen mit Sterilwasser entfernt. Nachteilig ist hierbei, dass durch das Spülen mit Sterilwasser viel Abwasser anfällt.

Aus der DE 196 42 987 A1 ist ein Verfahren zum Sterilisieren, Befüllen und Verschließen von Verpackungsbehältern bekannt. Hier ist es offenbart, Verpackungsbehälter mit Warmluft vorzuwärmen und anschließend ein Sterilisationsmittel, wie etwa Wasserstoffperoxid, einzuführen. Das Sterilisationsmittel kondensiert hierbei etwas. Nach der Sterilisation werden die Behälter getrocknet, um Reste des Sterilisationsmittels sicher herauszubekommen. Nachteilig ist hierbei, dass für die gesonderte Trocknung zum Entfernen des Sterilisationsmittels hohe Energiekosten anfallen.

Aus der DE 32 35 476 A1 ist ein Verfahren zum Entkeimen von Verpackungsmaterial bekannt, bei dem ein wasserstoffperoxidhaltiges Entkeimungsmittel zerstäubt, mit Druckluft gemischt auf die zu entkeimende Fläche des Verpackungsmaterials aufgeblasen und dort zum kondensieren gebracht wird. Hieraus ist es weiterhin bekannt, die zu entkeimende Fläche vor dem Aufblasen des Dampfluftgemisches auf eine Temperatur zu erwärmen, die der Taupunktstemperatur des Dampfluftgemisches entspricht oder etwas darunter liegt. Auch hier muss das entstehende Kondensat durch nachträgliches Auf- oder Einblasen von Heißluft wieder entfernt werden.

Die W002064174 A1 offenbart ein Verfahren zum Sterilisieren von vorgefertigten Bechern mit H2O2, bei dem die Becher in einer beheizten Sterilisationskammer von innen mit H2O2 beaufschlagt werden. Auch die Kammer selbst ist mit H2O2 beaufschlagt, sodass sich die Becher während des Transports durch die Kammer ständig in einer definierten H2O2 Atmosphäre befinden. Außerdem wird eine Steuereinrichtung offenbart, die mehrere Sensoren aufweist, um den H2O2 Gehalt an den verschiedenen Stellen im Sterilisationsprozess zu messen und die Sterilisation dadurch zu steuern.

Die DE4305478 A1 offenbart ein Verfahren zum Sterilisieren von Verbundpackungen mit sterilisierendem Gas, wobei der Gasverbrauch reduziert wird, indem die Verbundverpackung vor der Behandlung auf eine Temperatur von 60°C erwärmt wird. Außerdem wird das Sterilisationsmittel mittels einer Lanze im Bodenbereich der Verpackung aufgebracht, sodass eine Sterilisationsmittelströmung vom geschlossenen Ende der Verpackung hin zum offenen Ende entsteht.

Ein Verfahren zum Sterilisieren und Füllen mit stark sauren Produkten in einer Linearanlage offenbart die US 6120730. Dabei wird ein vorgeformter Behälter in einen beheizten Sterilisationstunnel mit stationären Düsen gebracht und unter diesen vorbeigefahren, sodass zum einen durch die Temperatur im Sterilisationstunnel die Behälter geheizt werden und zum anderen Sterilisationsmittel in die Behälter eingedüst wird. In einem weiteren Schritt wird erhitzte Luft in die Behälter eingebracht, bevor an zwei Fülldüsen das zu verpackende Produkt in die Behälter gebracht wird.

US 6692684B1 zeigt ein Verfahren zum Herstellen, füllen und verschließen von innen sterilen Behältern aus Vorformlingen, die in einem ersten Schritt über den Taupunkt eines gasförmigen Sterilisationsmittels erwärmt werden, um dann in einem zweiten Schritt mit Sterilisationsmittel beaufschlagt zu werden. Danach erfolgt eine weitere Erwärmung auf Umformungstemperatur, sodass der Vorformling unter Einwirkung von Hochdruck zu einem fertigen Behälter geformt werden kann. Nach der Formung es Behälters erfolgt ein Füllen und Verschließen.

Die US 6024917 offenbart ein um eine horizontale Achse umlaufendes Sterilisationskarussell mit mehreren nebeneinander und vielen umfänglich angeordneten Behandlungsstationen. Die einzelnen Behandlungsstationen sind verschließbar, sodass jeweils abgeschlossene Behandlungsräume entstehen. Das Sterilisationskarussell ist dabei mit Medienführungen derart ausgestattet, dass die einzelnen Stationen mit verschieden beaufschlagt werden können, wie z.B. mit H2O2, Sterilluft oder Vakuum. Dadurch wird eine extrem platzsparende Sterilisation von vielen Behältern ermöglicht.

Weiterhin offenbart US 6119433 ein System und ein Verfahren zum Herstellen von sterilen Plastikflaschen, wobei das System bei der Herstellung von Vorformlingen aus Kunststoffrohmaterial beginnt. Die Vorformlinge werden bereits in einem Reinraum hergestellt und anschließend zu fertigen Flaschen geblasen. Danach erfolgen ein Füllen mit einem sterilen Produkt und ein Verschließen mit zuvor sterilisierten Verschlüssen. Alle Schritte finden dabei in der gleichen sterilen Umgebung statt, sodass eine Rekontamination möglichst vermieden wird.

Aufgabe der Erfindung ist es, eine Vorrichtung und ein Verfahren zum Sterilabfüllen zu schaffen, das mit möglichst wenig Energie und/oder möglichst wenig Abwasseranfall auskommt und das eine möglichst schnelle Sterilisation erlaubt.

Diese Aufgabe wird gelöst durch eine Vorrichtung nach Anspruch 1 und ein Verfahren nach Anspruch 19. Bevorzugte Ausführungsformen sind in den jeweiligen Unteransprüchen offenbart.

Die Vorrichtung umfasst einen Sterilisator zum Sterilisieren der Flaschen mit H₂O₂. Weiterhin sind eine Einrichtung zum Befüllen und eine Einrichtung zum Verschließen der Flaschen vorgesehen. Durch das Verschließen ist das Füllgut der Flasche hermetisch gegen Verunreinigung geschützt und kann somit in eine unsterile Umgebung abgegeben werden.

Weiterhin sind Mittel vorgesehen, mit denen die Temperatur der Flaschen so eingestellt wird, dass das H₂O₂ auf den Flaschen nicht kondensiert. Während im Stand der Technik eine Kondensation des H₂O₂ für eine gute Sterilisation vorgesehen war, wird hier das H₂O₂ den Flaschen bei einer solchen Temperatur zugeführt, dass das H₂O₂ nicht kondensiert, sondern gasförmig bleibt. Auch hierdurch ergibt sich eine ausreichende Sterilisationswirkung. Ein Auswaschen oder übermäßig energieverbrauchendes Trocknen der Flaschen wird dabei jedoch vermieden.

Durch eine Temperatur, die oberhalb des Taupunkts liegt, ist es auch möglich eine so erhöhte Temperatur zu wählen, bei der durch die thermisch erhöhten Reaktionsraten eine sehr schnelle Sterilisation möglich ist.

Mit der Vorrichtung können beispielsweise PET-Flaschen sterilisiert werden, die üblicherweise mit einer Formblasmaschine, insbesondere einer Streckblasmaschine, aus Vorformlingen hergestellt werden. Die Formblasmaschine kann dabei mit dem Sterilisator über einen Förderer oder ein Übergabeeinrichtung verbunden sein. Jedoch können die Flaschen auch unabhängig von dem Sterilisator hergestellt worden sein.

Vorteilhaft ist es, die Temperatur der Flaschen durch entsprechende Kühlmittel in der Blasformmaschine einzustellen. Zum Formblasen werden die Vorformlinge vorgeheizt, sodass das Material des Vorformlings deformierbar wird. Die so hergestellten Flaschen sind nach dem Formblasprozess heiß und werden in der Formblasmaschine beispielsweise durch Einblasen von Kühlluft oder Verwendung wassergekühlter Formen gekühlt. Hierbei werden die Flaschen üblicherweise auf Temperaturen von 20° bis 30° abgekühlt. Durch ein weniger starkes Kühlen kann die Temperatur der ausgegebenen Flaschen jedoch auch erhöht sein, sodass die Flaschen aus der Formblasmaschine mit einer Temperatur von wenigstens 50°C wie etwa beispielsweise zwischen 50° und 60° ausgegeben werden. Bei entsprechendem H₂O₂-Druck oder H₂O₂-Partialdruck reicht dies aus, eine Kondensation von H₂O₂ in oder auf den Flaschen zu verhindern. Die benötigte Temperatur der Flaschen hängt auch von der Temperatur des H₂O₂ ab. Je niedriger die Temperatur des H₂O₂ ist, desto höher muss die Temperatur der Flaschen sein, da kaltes H₂O₂ durch eine leichte Kühlwirkung die Oberflächentemperatur der Flaschen absenken kann.

Es kann vorteilhaft sein, die Blasformmaschine und den Sterilisator nicht unmittelbar nebeneinander anzuordnen, um so die Zugänglichkeit zu beiden Einrichtungen zu erhalten. Dann ist es jedoch nötig, einen Zwischenförderer zwischen Blasmaschine und Sterilisator zwischenzuschalten. Dies kann eine einfache Gleitschiene sein, an der die Flaschen an ihrem Kragen hängend entlang oder herabgleiten oder auch ein Förderer mit einem Förderband, ein Luftförderer, ein Förderer mit Greifern oder Ähnliches.

Der Weg, den die Flaschen zwischen der Blasmaschine und dem Sterilisator zurücklegen, ist hierbei vorteilhafterweise thermisch isoliert. Dies kann durch eine einfache Ummantelung des Weges beispielsweise mit Plexiglasscheiben, Glas, Edelstahl oder Ähnlichem geschehen, die lediglich dafür sorgt, dass die warme Luft, die aus der Blasformmaschine austritt bzw. die durch die warmen Flaschen erzeugt wird, im Bereich der Flaschen gehalten wird. Die Ummantelung verhindert einen Wärmeverlust durch Konvektion. Zusätzlich können auch wärmeisolierende Materialien zur noch besseren thermischen Isolation eingesetzt werden. Durch die Ummantelung bzw. Isolation kann der Wärmeverlust der Flaschen verringert werden, so dass sie auch nach längerer Transportzeit noch eine genügend hohe Temperatur haben, um eine H₂O₂-Kondensation zu vertneiden.

Auf dem Weg, den die Flaschen zu dem Sterilisator durchlaufen, ist auch eine Heizeinrichtung vorgesehen. Damit ist es möglich, unabhängig von einer Formblasmaschine warme Flaschen für die Sterilisation zur Verfügung zu stellen.

Zum Aufwärmen der Flaschen kann eine Düse vorgesehen sein, mit der warme Luft in die Flaschen geblasen wird, um die Flaschen von innen aufzuwärmen. Die warme Luft wird vorzugsweise mit heißem Wasserdampf erzeugt, da dies schnell und kostengünstig ist. Dabei kann der Dampf z. B. überhitzt sein und mit der Luft vermischt werden. Auch kann die Luft in einem Wärmetauscher erhitzt werden. Die Luft hat vorzugsweise eine Temperatur von 100°C bis 150°C. Je höher die Temperatur, desto schneller kann die Aufwärmung der Flaschen erfolgen. Bei zu hohen Temperaturen kann sich eine PET-Flasche jedoch verformen, so dass bei besonders empfindlichen Flaschen auch mit einer Lufttemperatur von ca. 70°C gearbeitet werden kann.

Zum Aufwärmen kann auch eine Außenheizung vorgesehen sein, die die Flaschen von außen mit Heißluft anströmt. Diese Luft hat vorzugsweise eine Temperatur von 50° bis 60°C. Noch höhere Temperaturen der Außenluft benötigen mehr Energie und führen zu stark erhöhten Wärmeverlusten, da die Außenluft mit kälteren Maschinenteilen in Kontakt steht. Da aber PET ein Kunststoff ist, der keine gute Wärmeleitung zeigt, ist es vorteilhaft, die Flaschen nicht nur von innen, sondern auch von außen zu erwärmen, da so kürzere Aufwärmzeiten erreicht werden.

Um die Wärmeverluste zu minimieren, kann ein Tunnel vorgesehen sein, in dem die Heizeinrichtung die Flaschen aufheizen kann.

Vorzugsweise ist eine Düse oder ein Gasauslass vorgesehen, mit der H₂O₂ in Gasform oder als H₂O₂ -Luftgemisch in die Flaschen eingeblasen oder eingelassen werden kann. Mit Düsen lässt sich H₂O₂ so in den Flaschenhohlraum einblasen, dass es sich darin gut verteilt und alle Bereiche des Flascheninneren gut begast werden. Die Düse oder der Gasauslass ist vorzugsweise in die Flasche eintauchbar, um so eine bessere Konzentration des H₂O₂ in der Flasche zu erreichen.

Der Düse oder dem Gasauslass kann ein Lufterhitzer vorgeschaltet sein, der die Luft für die Düse oder den Gasauslass vorwärmt. Stromauf oder stromab des Lufterhitzers kann eine H₂O₂-Eindüsung vorgesehen sein. So kann ein vorgewärmtes H₂O₂-Luftgemisch erzeugt werden, mit dem die Sterilisation gut und schnell möglich ist.

Weiterhin ist vorteilhafterweise eine Düse oder ein Gasauslass vorgesehen, mit dem ein Sterilgas, wie beispielsweise Sterilluft, in die Flaschen eingeleitet werden kann, um so das H₂O₂ wieder auszublasen. Hierbei ist vorzugsweise eine Vorwärmeinrichtung zum Vorwärmen des Sterilgases vorgesehen, um so zu verhindern, dass das H₂O₂ durch das Einblasen von kalter Luft kondensiert. Bei genügend hoher Flaschentemperatur kondensiert das H₂O₂ jedoch auch nicht auf den Flaschen bei Einblasen von nicht vorgewärmter Sterilluft.

Vorzugsweise sind Mittel vorgesehen, mit denen auch die Außenseite der Flaschen sterilisiert werden kann. Dies kann z. B. durch einen Sterilisationstunnel gegeben sein, der aus den Flaschen austretendendes H₂O₂ bei der Außenseite der Flaschen hält. Auf diese Weise kann das aus dem Flascheninneren verlorene H₂O₂ für die Außensterilisation gewonnen werden und so der H₂O₂-Verbrauch reduziert werden. Um eine geeignete H₂O₂-Konzentration außerhalb der Flaschen sicherzustellen, können zusätzliche H₂O₂-Luft-Gemischanschlüsse vorgesehen sein. Um eine geeignete Temperatur in dem Sterilisationstunnel zu erreichen, können sowohl Warm- als auch Kaltluftanschlüsse vorgesehen sein. Bei der geeigneten Temperatur kondensiert das H₂O₂ nicht in oder auf den Flaschen.

Stromauf des Sterilisators kann ein Warmhaltetunnel vorgesehen sein, mit dem die erwärmten Flaschen auf der gewünschten Temperatur gehalten werden. Dadurch kann sichergestellt werden, dass die Flaschen auf dem Weg von der Heizeinrichtung oder einer Formblasmaschine zum Sterilisator nicht abkühlen, sondern vielmehr die gewünschte Temperatur beibehalten. Diese Strecke kann durch eine H₂O₂-haltige Atmosphäre auch zur Außenbehandlung genutzt werden. Dadurch wird die Außensterilisation noch weiter verbessert.

Vorteilhafterweise werden auch die Flaschenverschlüsse mit H₂O₂ sterilisiert. Um auch hier eine Kondensation des H₂O₂ auf den Flaschenverschlüssen zu vermeiden, ist eine Vorwärmeinrichtung vorgesehen, mit der die Flaschenverschlüsse auf eine Temperatur oberhalb des Taupunkts von H₂O₂ gebracht werden können. Dies kann durch Beaufschlagungen mit Heißluft oder Infrarotbestrahlung geschehen. Vorteilhaft ist es weiterhin, wenn die Flaschenverschlüsse in einem Speicher sterilisiert werden, da dann die Einwirkzeit des H₂O₂ möglichst groß ist.

Vorzugsweise sind weiterhin Mittel vorgesehen, die den Sterilisator insgesamt thermisch isolieren. Dazu kann eine Ummantelung mit einfachen Trennwänden aus Edelstahl, Plexiglas, Glas oder Ähnlichem vorgesehen sein. Es können jedoch auch zusätzlich thermisch isolierende Materialien eingesetzt werden. Durch die Ummantelung wird erreicht, dass die gewärmte Luft bei dem Sterilisator verbleibt. Durch die Aufwärmung des gesamten Sterilisators auf eine Temperatur oberhalb des Taupunkts von H₂O₂ wird verhindert, dass das H₂O₂ an anderen Stellen kondensiert. Dies wäre für die Sterilisation der Flaschen an sich nicht schädlich, führt aber zu einem erhöhten H₂O₂ -Verbrauch und dazu, dass das H₂O₂ eventuell flüssig abläuft, was nicht wünschenswert ist.

Zusätzlich kann auch eine Beheizung vorgesehen sein, mit der der gesamte Sterilisator beheizt werden kann. Dies kann durch ein Heißluftgebläse, elektrische Heizelemente oder sonstige Einrichtungen geschehen. Dadurch lässt sich gut eine komplette und ausreichend gleichmäßige Aufwärmung des Sterilisators erreichen.

Weiterhin kann vorgesehen sein, weitere Flaschen- oder Verschlussbehandlungsgeräte thermisch durch Ummantelung und/oder Wärmedämmung zu isolieren oder zusätzlich zu beheizen. Derartige Flaschen oder Verschlussbehandlungsgeräte können beispielsweise ein Flaschenspeicher sein, wie bevorzugterweise ein dynamischer Flaschenspeicher. Dadurch kann erreicht werden, dass die Flaschen auf dem Weg zwischen der Formblasmaschine oder einer Heizeinrichtung und dem Sterilisator nicht zu stark abkühlen.

Zum Beheizen der verschiedenen Einrichtungen kann beispielsweise die Heißluft verwendet werden, die in der Blasformmaschine oder bei dem Aufheizen der Vorformlinge anfällt. Diese Heißluft kann beispielsweise zum Beheizen eines Flaschenspeichers, des Sterilisators, des Weges zwischen Blasmaschine und Sterilisator oder Ähnlichem eingesetzt werden. Eventuell können Zusatzheizungen vorgesehen sein, falls die Heißluft, die bei dem Formblasvorgang oder dem Vorwärmen der Vorformlinge anfällt, nicht ausreicht. Dies kann insbesondere beim Hochfahren der Maschine relevant sein.

Beim erfindungsgemäßen Verfahren werden Flaschen mit H₂O₂ sterilisiert sowie anschließend steril befüllt und verschlossen. Beim Sterilisieren haben die Flaschen eine solche Temperatur, die eine Kondensation des H₂O₂ in oder an den Flaschen verhindert. Die Flaschen haben durch den Formblasvorgang die benötigte Temperatur. Sie werden auch durch Heißluft auf die nötige Temperatur gebracht. Weiterhin ist es vorteilhaft, die Flaschen nach dem Formblasvorgang thermisch isoliert, d. h. ummantelt oder mit Wärmedämmmaterialien umgeben, zu fördern, um so ein zu starkes Abkühlen der Flaschen zu vermeiden.

Vorteilhafterweise wird die Sterilisation durch Einblasen von H₂O₂ und durch Ausblasen desselben, beispielsweise mit Sterilluft vorgenommen. Dies führt zu einer raschen und ausreichend starken Sterilisation. Dadurch, dass das Entfernen des H₂O₂ relativ einfach möglich ist und somit nur wenig Zeit benötigt, kann die Einwirkzeit des H₂O₂ erhöht werden, ohne dass die Gesamtverweilzeit der Flaschen in dem Sterilisator im Vergleich zum Stand der Technik erhöht würde, da weniger Zeit zum Entfernen des H₂O₂ benötigt wird im Vergleich zu konventionellen Verfahren.

Bei dem Verfahren wird der Sterilisator vorzugsweise insgesamt oder teilweise auf eine solche Temperatur geheizt, dass diejenigen Teile, die mit H₂O₂ in Kontakt kommen, eine Temperatur oberhalb des Taupunkts von H₂O₂ haben.

Besonders vorteilhaft ist ein Verfahren, bei dem zwischen der Formblasmaschine und dem Sterilisator ein Speicher für Flaschen vorgesehen ist, der vorzugsweise ein dynamischer Speicher ist, der nach dem FI-FO (first in - first out) Prinzip arbeitet. Damit kann der Formblasvorgang bei einem Ausfall des Sterilisators/Füllers weiter betrieben werden und die hergestellten Flaschen in dem Speicher zwischengespeichert werden. Der Zwischenspeicher ist hierzu vorzugsweise so ausgebildet, dass die Flaschen in dem Speicher nicht oder nicht sehr stark abkühlen, sondern vielmehr auf Temperatur gehalten oder noch weiter aktiv aufgewärmt werden. Dadurch können auch gespeicherte Flaschen ohne Kondensation von H₂O₂ sterilisiert werden. Im übrigen wird durch die Aufrechterhaltung der Temperatur der Flaschen aus dem Blasformvorgang verhindert, dass zusätzliche Energie zum Beheizen der Flaschen benötigt wird, was zu einer Energieersparnis führt.

Die an den verschiedenen Stellen eingesetzte Luft, wie beispielsweise die Luft, die mit H₂O₂ angereichert wird, die Luft zum Ausblasen oder die Luft zum Heizen ist bevorzugterweise ionisierte Luft. Die ionisierte Luft wird in einem Ionisator durch Hochspannung hergestellt und erleichtert die Entfernung von Partikeln.

Mehrere Ausführungsformen der Vorrichtungen und des Verfahrens werden anhand der Zeichnungen erläutert. Dabei zeigt:
- Figur 1: eine schematische Darstellung einer Vorrichtung,
- Figur 2: eine schematische Darstellung einer Vorrichtung mit einem Speicher, und
- Figur 3: eine schematische Darstellung einer Vorrichtung mit einer eigenen Heizeinrichtung gemäß der Erfindung.

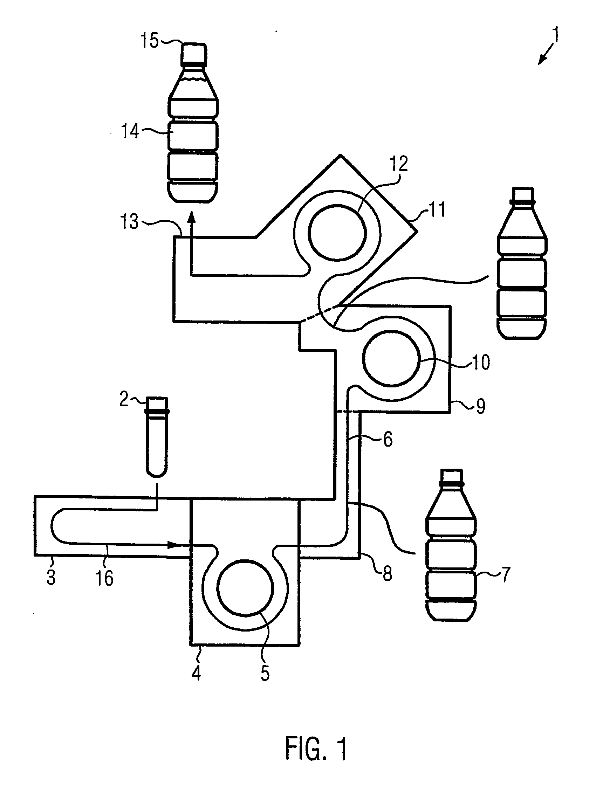

In Figur 1 ist eine Vorrichtung 1 mit einer Blasmaschine 4 in Form einer Streckblasmaschine, einem Sterilisator 9 und einem Füller und Verschließer 11 gezeigt. Die Blasmaschine 4 kann über ein Karussell 5 verfügen, an dem die Blasformen umlaufen. Der Blasmaschine 4 ist eine Heizstation 3 mit einer Förderkette 16 vorgeschaltet, in der Vorformlinge 2 aus PET auf Verarbeitungstemperatur erwärmt werden.

Die Kühlung der Blasformen ist derart eingeregelt, dass die fertigen Flaschen 7 die Blasmaschine 4 mit einer Temperatur von ca. 50 bis 60°C verlassen.

Stromab der Blasmaschine 4 ist eine Wegstrecke 6 zwischen der Formblasmaschine 4 und dem Sterilisator 9 dargestellt. Entlang der Wegstrecke 6 werden fertig ausgeformte Flaschen 7 transportiert. Hierzu kann ein entsprechend geeigneter Förderer (Förderer mit Greifern, Gleitschiene, Luftförderer etc.) vorgesehen sein. Der Förderer kann beispielsweise den Kragen am oberen Ende der Flaschen 7 unterhalb des Flaschengewindes ausnutzen, um die Flaschen zu halten.

Die Wegstrecke 6 ist in einem Tunnel 8 angeordnet, der die Wegstrecke 6 ummantelt. Der Tunnel 8 kann beispielsweise aus Edelstahl, Plexiglas, Glas oder einer Stahlglaskonstruktion oder Ähnlichem gebildet sein. Auch ist es möglich, dass der Tunnel 8 mit wärmeisolierendem Dämmmaterial umgeben ist.

An die Wegstrecke 6 schließt sich ein Sterilisator 9 an. Auch dieser kann über ein Karussell 10 verfügen, das mit entsprechenden Ein- und Ausgabesternen versehen ist. Entlang des Weges, den die Flaschen um das Karussell nehmen bzw. am Umfang des Karussells, sind Düsen vorgesehen, mit denen H₂O₂ in die Flaschen 7 eingeblasen werden kann. Weiterhin sind Düsen vorgesehen, mit denen Sterilluft in die Flaschen 7 eingeblasen wird, um das H₂O₂ auszublasen. Die Düsen zum Einblasen von H₂O₂ sowie die Düsen zum Einblasen von Luft können die selben Düsen sein, die dann jeweils an zwei entsprechende Zuleitungen für H₂O₂ und für die Sterilluft angeschlossen sind.

Das H₂O₂ wird vorteilhafterweise durch Verdampfen von H₂O₂ auf einer heißen Platte in einer separaten Vorrichtung (z.B. Flashverdampfer) erzeugt. Dazu kann es in flüssiger Form dosiert auf eine heiße Platte gegeben werden, z. B. durch Auftropfen. Das so gewonnene gasförmige H₂O₂ wird dann der Düse oder den Düsen durch entsprechende Leitungen zugeleitet.

Das H₂O₂ -Gas kann entweder in Reinform verwendet werden oder vermischt mit einem anderen Gas wie Luft, Stickstoff, Sauerstoff, Wasserdampf oder ähnlichem.

Bevorzugterweise wird ein H₂O₂-Heißluft-Gemisch verwendet. Das Gemisch hat bei der Abgabe beispielsweise eine Temperatur von 150°C. Durch die hohe Temperatur ergibt sich eine gute Sterilisationswirkung. Niedrigere Temperaturen sind aber auch möglich (Energieersparnis). Noch höhere Temperaturen könnten je nach Volumenstrom und Behandlungszeit zu Flaschenverformungen führen.

Die Düsen oder Gasauslässe für das Gemisch sind an einem Karussell angeordnet. Dabei wird normale Umgebungsluft gefiltert und evtl. getrocknet, über einen Drehverteiler dem drehenden Teil des Karussells 10 zugeführt. Dort wird das in einem Flashverdampfer erzeugte H₂O₂ in den Luftweg eingedüst. Stromauf oder stromab der Eindüsung wird die Luft bzw. das Luft- H₂O₂-Gemisch in einem Elektroerhitzer erhitzt. Ist der Erhitzer stromab der Eindüsung angeordnet, wird die Luft beispielsweise auf 200°C bis 300°C aufgeheizt, vorzugsweise 250°C bis 300°C. Für mehrere Düsen oder Gasauslässe kann ein gemeinsamer Flashverdampfer vorgesehen sein. In der Flasche kann dann die Temperatur entsprechend niedriger sein.

Das heiße Gemisch wird eine vorbestimmte Zeit lang, wie z. B. 2 Sekunden bis 10 Sekunden, vorzugsweise etwa 8 Sekunden, den Flaschen 7 zugeführt. Das H₂O₂ kondensiert nicht in den Flaschen. In der Behandlungszeit kann eine Reduktionsrate von bis zu Log 6 erreicht werden.

In den Zeiten, in denen eine Düse oder ein Gasauslass kein Gemisch in Flaschen einbringt, wird das erzeugte Gemisch über einen Bypass zur Flaschenaußenbehandlung abgeführt. Dies kommt z. B. vor, wenn bei der Düse keine Flasche 7 gehalten wird.

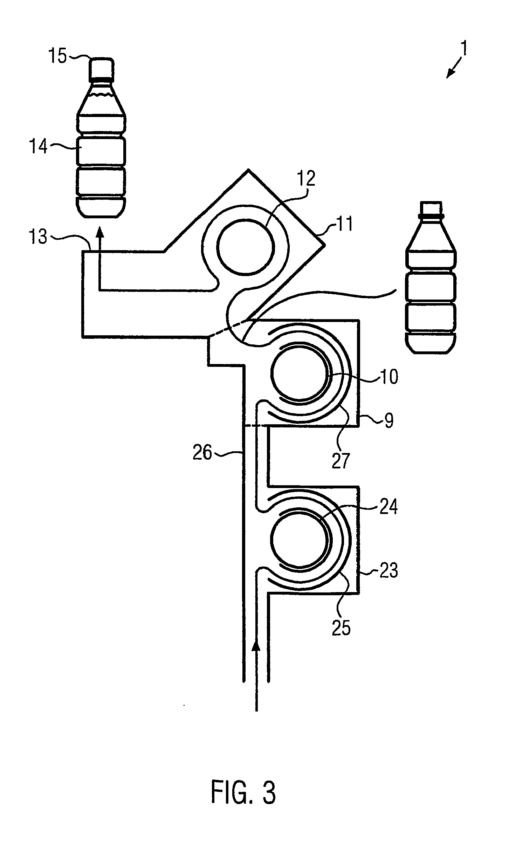

Zur Flaschenaußenbehandlung ist der Weg der Flaschen 7 um das Karussell 10 herum mit einem Tunnel (siehe Bezugsziffer 27 in Fig. 3) umgeben. Dadurch kann im Bereich des Flaschenwegs eine sterilisierende Atmosphäre geschaffen werden. Die Temperatur im Tunnel (im Folgenden zur Unterscheidung von anderen Tunneln Sterilisationstunnel genannt) kann durch Zufuhr von Heiß- oder Kaltluft auf ca. 50°C eingestellt werden. Bei dieser Temperatur kondensiert das H₂O₂ nicht. Bei Betrieb strömt etwas des H₂O₂ -Heißluftgemischs aus dem Flascheninneren aus den Flaschen heraus und reichert somit die Atmosphäre in dem Sterilisationstunnel mit H₂O₂ an. Zusätzlich kann H₂O₂ in den Tunnel (in Gas oder Gemischform) eingeleitet werden. Dies kann aus dem Bypass der Flascheninnenbehandlung kommen oder mit einer separaten Zuleitung für H₂O₂ und/oder heißem und/oder kaltem H₂O₂ -Luft-Gemisch zugeführt werden.

Um H₂O₂-Verluste aus dem Sterilisationstunnel heraus zu minimieren, ist der Tunnel möglichst gasdicht. Zwischen den beweglichen und feststehenden Teilen des Sterilisationstunnels sind vorzugsweise schleifende Dichtungen vorgesehen.

Unabhängig davon, ob ein Sterilisationstunnel vorgesehen ist oder nicht, kann der Sterilisator 9 ummantelt sein. Dies dient zum einen dazu, das H₂O₂-Gas nicht in die Umgebung gelangen zu lassen. Zum anderen soll die warme Luft bei dem Sterilisator 9 gehalten werden. Die Ummantelung des Sterilisators 9 kann durch entsprechende Verkleidungen oder Ähnliches erreicht werden. Am besten ist die Verkleidung gasdicht. Auch kann im Bereich des Sterilisators 9 ein leichter Unterdruck herrschen, damit durch eventuelle Undichtigkeiten in der Ummantelung kein H₂O₂ ausströmt, sondern vielmehr normale Luft eingesaugt wird. Auch dadurch wird ein Austreten von H₂O₂ -Gas vermieden.

Entlang des Weges, den die Flaschen 7 in den Sterilisator 9 hinein und aus diesem heraus nehmen, können beispielsweise nur kleine, gerade für die Flaschen 7 passende, Öffnungen vorhanden sein (in den Figuren bei den gestichelten Linien), um zwar einen Flaschentransport zu ermöglichen, jedoch die Ummantelung des Sterilisators 9 bzw. des Sterilisationstunnels zu gewährleisten. Auch können Schleusen vorgesehen sein, um die Flaschen 7 in den Sterilisator 9 hinein- und wieder herauszuschleusen.

Stromab des Sterilisators 9 ist ein Füller und Verschließer 11 angeordnet. Auch dieser weist ein Karussell 12 auf, an dessen Umfang Füllventile zum Befüllen der Flaschen 7 mit Füllgut 14 in Form eines sterilisierten Getränks vorgesehen sind. Weiterhin können die Flaschen 7 mit Verschlusskappen 15 verschlossen werden. Der Füller und Verschließer 11 ist ebenfalls ummantelt, um sterile Bedingungen beim Abfüllen und Schließen zu gewährleisten. Die so verschlossenen Flaschen 7 können aus der Vorrichtung 1 beim Ausgang 13 ausgegeben werden. Das Verschließen der Flaschen 7 kann sowohl auf dem Füllerkarussell 12 erfolgen, als auch in einem stromab des Füllers angeordneten Verschließer.

Zur Sterilisation der Flaschenverschlüsse 15 werden diese in einem Speicher auf einer Temperatur von 45°C bis 60°C gehalten und dort mit H₂O₂ begast. Zwischen Speicher und Verschließer ist eine Transporteinrichtung für die Flaschenverschlüsse vorgesehen. Auf dem Weg zwischen Speicher und Verschließer sind Blasdüsen angeordnet, mit denen das H₂O₂ aus den Verschlüssen entfernt werden kann. Weiter kann die Temperatur der Flaschenverschlüsse mit dem Ausbläser selber oder mit einer anderen Kühleinrichtung stromauf des Verschließers gesenkt werden, um die Formstabilität der Flaschenverschlüsse zu gewährleisten.

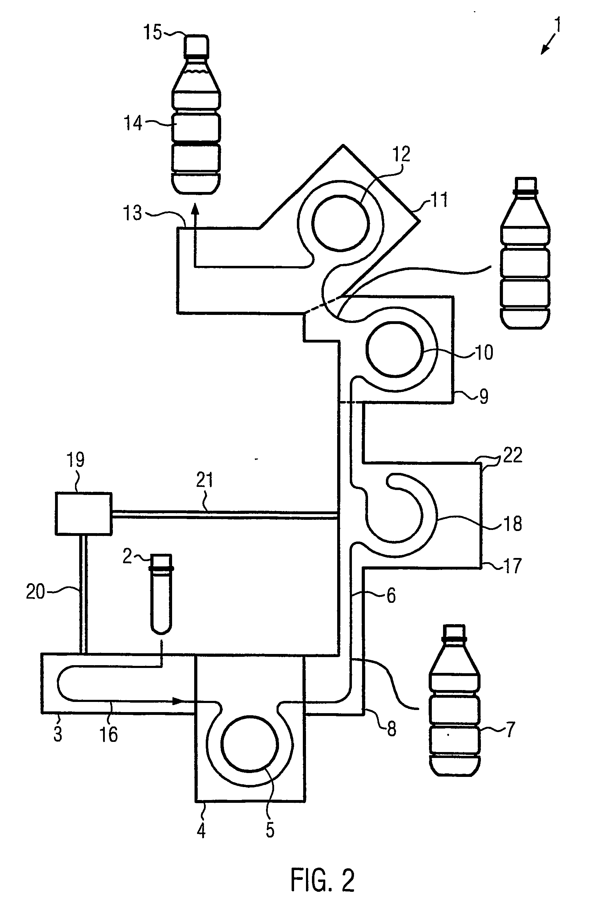

In Figur 2 ist zu den Vorrichtungen aus Figur 1 zusätzlich ein Speicher 17 zwischen der Blasmaschine 4 und dem Sterilisator 9 angeordnet. Dieser Speicher 17 kann kurzfristige Kapazitätsunterschiede zwischen der Blasmaschine 4 und dem Sterilisator 9 bzw. dem Füller 11 ausgleichen. Beispielsweise bei Stillstand der Sterilisation oder der Abfüllung kann die Blasmaschine 4 Flaschen weiterproduzieren, die in den Speicher 17 aufgenommen werden. Diese können dann anschließend durch eine Beschleunigung des Sterilisierens/Abfüllens und/oder des Verlangsamens der Blasmaschine 3 abgebaut werden. Auch kann bei Ausfall der Blasmaschine 4 der Speicher 17 ganz oder teilweise entleert werden, um die Sterilisation und das Abfüllen kontinuierlich weiterzuführen, so lange, bis die Blasmaschine 4 wieder Flaschen 7 nachliefern kann.

Der Speicher 17 umfasst vorteilhafterweise Wendelbahnen 18, entlang denen die Flaschen transportiert werden, wobei die Länge der Wendelbahn 18, die die Flaschen 7 in dem Speicher 17 durchlaufen, variabel ist. Dadurch ergibt sich ein dynamischer Flaschenspeicher 17.

Der Flaschenspeicher 17 ist mit einer Wandung 22 gekapselt, um die warme Luft, die aus der Formblasmaschine 4 austritt oder die durch die Wärme der Flaschen 7 erzeugt wird, bei den Flaschen 7 zu halten. Die Wandung 22 kann beispielsweise aus Metall, Kunststoff, wie Plexiglas, Glas oder Ähnlichem aufgebaut sein, um die warme Luft bei den wendelförmigen Bahnen 18 einzukapseln. Die Wandungen 22 des Speichers 17 können auch wärmedämmendes Material umfassen.

Der Innenraum des Speichers 17 kann auch mit Warmluft beheizbar sein. Hierzu kann normale Luft aufgewärmt oder aber auch die Abluft aus dem Blasformvorgang verwendet werden. In der Heizeinrichtung 3 entsteht beispielsweise durch das Aufheizen der Vorformlinge 2 Warmluft, die über eine Leitung 20 einer Steuer- oder Regeleinheit 19 zugeführt werden kann, die kontrolliert Warmluft über eine Leitung 21 in den Speicher 17 einbläst. Der Speicher 17 muss dann auch über einen entsprechenden Luftauslass verfügen. Die Leitung 21 kann auch in dem Bereich der Wegstrecke 6 enden. In der Einheit 19 oder stromauf/stromab von dieser in den Leitungen 20, 21, kann auch noch eine zusätzliche Heizeinrichtung vorgesehen sein, um Luft aufzuheizen, falls in der Vorwärmeinrichtung 3 nicht oder nicht genügend warme Luft erzeugt wird. Die Leitung 20 kann auch von der Blasmaschine 4 abzweigen oder eine zusätzliche Zuleitung von der Blasmaschine 4 zu Steuer- und Regeleinrichtung 19 umfassen.

Die Ummantelungen bzw. Verkleidungen der verschiedenen jeweils benachbarten Einrichtungen wie des Wegs 6, des Speichers 17, des Sterilisators 9 und des Füllers 11 können auch gemeinsam vorgesehen sein. In der Figur 2 ist beispielsweise die linke Verkleidung bzw. Ummantelung des Wegs 6, des Speichers 17 und des Sterilisators 9 zusammenhängend dargestellt.

In Figur 3 ist eine Ausführungsform gemäß der Erfindung gezeigt, bei der die Flaschen 7 mit einer Heizeinrichtung 23 auf die gewünschte Temperatur gebracht werden. Die Heizeinrichtung 23 umfasst ein Karussell 24, an dem die Flaschen 7 umlaufen können. Am Umfang des Karussells 24 sind Düsen angeordnet, mit denen heiße Luft in die Flaschen geblasen werden kann. Die Luft hat eine Temperatur von ca. 100°C bis 150°C. Zum Heizen der Luft ist eine Dampfzufuhr vorgesehen, mit der heißer Dampf mit der Luft gemischt werden kann, um so die Luft zu aufzuwärmen. Auch kann zur Lufterhitzung ein Wärmetauscher oder ein sonstiger Lufterhitzer vorgesehen sein. Die Luft hat eine solche Feuchtigkeit, dass sich in den Flaschen kein Kondensat bildet. Die ungewärmte Luft und der Dampf werden dem drehenden Teil vorzugsweise über einen jeweils separaten Drehverteiler zugeleitet. Die Lufterhitzung erfolgt erst am drehenden Teil des Karussells. Dadurch kann die Luft erst unmittelbar vor der Ausgabe erhitzt werden, so dass sie nicht auf dem Weg zur Abgabe wieder abkühlen kann, was zu Energieverlusten führen würde.

Die Heißluftleitung hat weiter einen Bypass, der die heiße Luft an den Düsen vorbei in den Tunnel 25 leitet. Der Tunnel 25 dient dazu, die warme Luft bei dem Weg der Flaschen 7 zu halten, damit eine Außenwärmung erreicht wird. Zum Einstellen der Temperatur in dem Tunnel 25 kann noch zusätzlich ein oder mehrere Warm- oder Kaltluftanschlüsse vorgesehen sein. Zur Warmlufterzeugung sind beispielsweise Heizpatronen vorgesehen, die sich kurz vor dem Lufteinlass in den Tunnel 25 befinden. Die Heizung ist so ausgelegt, dass in dem Tunnel eine Lufttemperatur von 50°C bis 60°C erreicht wird. Dies entspricht der Temperatur, auf die die Flaschen gebracht werden sollen.

In den Figuren 1 bis 3 ist stromauf des Sterilistors 9 eine Wegstrecke, die die Flaschen zurücklegen müssen, um zu dem Sterilisator zu gelangen (siehe z. B. Weg 6 in Fig. 1 und 2 sowie Weg 26 in Figur 3). Diese Strecke kann durch einen Förderer oder durch einen oder mehrere Transfersterne vorgegeben sein. Die Strecke ist vorzugsweise in einem Tunnel 8, 26 angeordnet (im Folgenden zur Unterscheidung Warmhaltetunnel genannt). In den Warmhaltetunnel 8, 26 kann z.B. aus dem Sterilisator 9 warme Luft, die H₂O₂-haltig ist, eintreten. Zusätzlich können Anschlüsse für H₂O₂ und/oder heißes und/oder kaltes H₂O₂ -Luft-Gemisch an dem Warmhaltetunnel 8, 26 vorgesehen sein. Bevorzugterweise wird ein Luftstrom in dem Warmhaltetunnel erzeugt, der der Bewegungsrichtung der Flaschen entgegengesetzt ist (Gegenstromverfahren). Dazu kann eine Absaugeinrichtung am Flascheneintritt des Warmhaltetunnels vorgesehen sein, die die H₂O₂ -haltige Luft aus dem Sterilisator durch den Warmhaltetunnel zieht und dann wegführt. Der Warmhaltetunnel 8, 26 ist vorzugsweise mit dem Sterilisationstunnel 27 dicht verbunden, so dass kein H₂O₂ oder heiße Luft an dem Übergang entweichen kann. Auch ist die Verbindung zwischen dem Tunnel 25 und dem Warmhaltetunnel 26 vorzugsweise gasdicht.

Das Verfahren soll anhand von Figuren 1 bis 3 erläutert werden. Vorformlinge 2 werden in eine Heizstation 3 eingebracht, in der sie erwärmt werden. Die Verarbeitungstemperatur der Vorformlinge liegt oberhalb von 120° Celsius. Der Weg der Vorformlinge 2 bzw. der Weg der Flaschen 7 mit der Förderkette ist mit der Linie 16 schematisch dargestellt. Die erwärmten Vorformlinge 2 werden in die Blasmaschine 4 überführt und dort zu Flaschen 7 geblasen. Die Flaschen 7 weisen nach dem Streckblasvorgang eine erhöhte Temperatur auf, die sich noch aus der Erwärmung der Vorformlinge in der Heizstation 3 ergibt. Üblicherweise werden die Flaschen auf eine möglichst niedrige Temperatur abgekühlt. Bei dem vorliegenden Verfahren werden die Flaschen jedoch nicht auf die tiefstmögliche Temperatur abgekühlt, sondern vielmehr bei einer Temperatur von 50° bis 70°, vorzugsweise zwischen 50° und 60° belassen.

Die Flaschen 7 werden mit einer solchen Temperatur in den Tunnel 8 abgegeben, dass sie nach Durchlauf desselben noch eine ausreichend hohe Temperatur haben. Der Tunnel 8 ist dazu so ausgestaltet, dass die Flaschen 7 nur wenig oder keine Wärme verlieren, um so die ausreichend hohe Temperatur beizubehalten.

Wie in Figur 3 gezeigt, sind die Flaschen 7 auch mit einer Heizeinrichtung 23 auf die benötigte Temperatur gebracht. Dazu wird in die Flaschen 7 heiße Luft eingeblasen. Die Luft wird hierfür mit Dampf, einem Wärmetauscher, einem Elektroerhitzer oder ähnlichem auf eine Temperatur von 100°C bis 150°C erhitzt. Die Heißlufteinleitung dauert ca. 3 bis 7 Sekunden, vorzugsweise etwa 5 Sekunden.

Weiter werden die Flaschen 7 von außen beheizt. Dazu werden die Flaschen 7 in einem Tunnel 25 transportiert, in dem eine Lufttemperatur von ca. 40 bis 60 °C vorherrscht. Die Temperatur in dem Tunnel 25 ergibt sich zum einen durch die Heißlufteinleitung in die Flaschen 7 und durch heiße Luft, die durch den Bypass an den Heißluftdüsen vorbei in den Tunnel 25 gelangt. Zu den Zeiten, zu denen keine Heißluft in die Flaschen geleitet werden soll, strömt die Heißluft durch den Bypass in den Tunnel 25. Dadurch kann die Heißluftgewinnung kontinuierlich betrieben werden, die Flaschen 7 werden aber nur eine definierte Zeit lang der heißen Luft direkt ausgesetzt.

Um in dem Tunnel 25 eine Temperatur von 50°C bis 60°C zu erhalten, wird zusätzlich mit Heizpatronen erwärmte Heißluft in den Tunnel 25 geblasen. Hierzu ist eine Temperatursteuerung vorgesehen. Anschließend werden die Flaschen 7 in den Tunnel 26 (Warmhaltetunnel) überführt. Dort herrscht eine Temperatur von 50°C bis 60°C, so dass die Flaschen 7 nicht auskühlen, sondern vielmehr auf der gewünschten Temperatur gehalten werden. Weiter herrscht dort eine H₂O₂ -haltige Atmosphäre, so dass die Flaschen 7 zumindest außen vorsterilisiert werden. Nach Transport durch den Warmhaltetunnel 26 werden die Flaschen 7 in der Sterilisator 9 geführt.

Die Flaschen 7 werden mit einer ausreichend hohen Temperatur in den Sterilisator 9 eingebracht, so dass bei der Begasung mit H₂O₂ in dem Sterilisator 9 das H₂O₂ nicht kondensiert. Dazu wird zum einen ein heißes H₂O₂ -Luftgemisch (Temperatur 125°C bis 175°C, bevorzugt 150°C) in die Flaschen mit einer Düse eingeblasen und die Flaschen 7 in einer auf die Außenseite einwirkenden H₂O₂-haltigen Atmosphäre transportiert. Die äußere Atmosphäre hat eine Temperatur von ca. 50°C bis 60°C. Durch das Verhindern der Kondensation kann das gasförmige H₂O₂ daher einfach durch Einblasen von Sterilluft ausgeblasen werden und die so sterilisierten Flaschen werden gefüllt und verschlossen und anschließend ausgegeben.

Zum Verschließen werden Flaschenverschlüsse 15 sterilisiert. Dazu werden die Verschlüsse bei einer Temperatur von ca. 50°C bis 60°C in einem Speicher gehalten und dort H₂O₂ ausgesetzt. Die Verweilzeit kann mehrere Sekunden bis mehrere Minuten betragen. Anschließend werden die Verschlüsse aus dem Speicher abtransportiert und dabei das H₂O₂ von den Verschlüssen durch Abblasen und Absaugen entfernt. Auch werden die Verschlüsse hierbei abgekühlt, so dass sie formstabil und damit gut handhabbar sind. Die so sterilisierten und gekühlten Verschlüsse werden unter sterilen Bedingungen dem Verschließer zugeführt.

Nach Ausgabe der Flaschen 7 aus der Formblasmaschine 4 oder auch der Heizeinrichtung 23 können die Flaschen auch in einen Speicher 17 eingeführt werden, in dem sie entlang von Wendelbahnen transportiert werden, um so einen Speichereffekt zu erzielen. Die Länge der Wendelbahn ist hierbei variabel, um eine variable Puffergröße zu erhalten. Der Speicher 17 ist so ausgestaltet, dass die Flaschen 7 während der Speicherung wenig Wärme verlieren oder vielmehr auf ihre Temperatur gehalten werden. Dazu wird beispielsweise Warmluft aus der Vorwärmeinrichtung 3 über eine Zuleitung 20 zu einer Regel- und Steuereinheit 19 übertragen, die die Luft dann entlang eines Kanals 21 in den Innenraum des Speichers 17 leitet. Die Steuer- und Regeleinheit 19 stellt dabei eine Temperatur im Inneren des Speichers 17 her, die so hoch ist, dass H₂O₂ auf den Flaschen 7 während der Sterilisation in dem Sterilisator 9 nicht kondensiert.

## Patentansprüche

1. Vorrichtung zum Sterilabfüllen von Flüssigkeiten in Flaschen mit
- einem Sterilisator zum Sterilisieren der Flaschen mit H₂O₂,
- einem Füller zum Füllen der Flaschen und
- einem Verschließer zum Anbringen eines Verschlusses wie etwa einer Verschlusskappe, wobei Mittel vorgesehen sind, mit denen die Temperatur der Flaschen (7) so eingestellt werden kann, dass eine Kondensation des H₂O₂ auf der Flaschenoberfläche verhindert wird, wobei die Mittel Kühlmittel in einer Blasformmaschine (4), mit der die Flaschen hergestellt werden, umfassen, mit denen die Temperatur der Flaschen (7) bei Ausgabe aus der Blasformmaschine (4) eingestellt wird **dadurch gekennzeichnet, dass** die Mittel ferner eine Heizeinrichtung (23) für den Weg der Flaschen (7) zu dem Sterilisator (9) umfassen, wobei die Heizeinrichtung (23) eine Innenheizeinrichtung umfasst, mit der vorgewärmte Luft in die Flaschen (7) geblasen werden kann.

2. Vorrichtung nach Anspruch 1, **dadurch gekennzeichnet, dass** die Mittel eine Ummantelung und/oder eine thermische Isolation des Weges (6) der Flaschen (7) zwischen Blasmaschine (4) und Sterilisator (9) umfassen.

3. Vorrichtung nach Anspruch 1, **dadurch gekennzeichnet, dass** zum Vorwärmen der Luft eine Dampfzufuhr vorgesehen ist, mit der die Luft auf eine Temperatur oberhalb der gewünschten Temperatur der Flaschen (7) gebracht werden kann.

4. Vorrichtung nach Anspruch 1, **dadurch gekennzeichnet, dass** die Heizeinrichtung (23) eine Außenheizeinrichtung umfasst, mit der die Flaschen (7) von außen beheizt werden können, wobei die Außenheizeinrichtung ein Heißluftgebläse umfasst, mit dem die Flaschen (7) von außen angeströmt werden können.

5. Vorrichtung nach Anspruch 1, **dadurch gekennzeichnet, dass** die Heizeinrichtung (23) einen Tunnel (25) um den Flaschenweg umfasst.

6. Vorrichtung nach einem der Ansprüche 1 bis 5, **dadurch gekennzeichnet, dass** mindestens eine Düse oder mindestens ein Gasauslass zum Einbringen von H₂O₂ in Gasform in die Flaschen (7) vorgesehen ist, wobei die Düse oder der Gasauslass vorzugsweise so gelagert ist, dass sie/er in die Flasche eingetaucht werden kann.

7. Vorrichtung nach Anspruch 6, **dadurch gekennzeichnet, dass** als Lufterhitzer ein Wärmetauscher und/oder ein Elektroerhitzer, vorgesehen ist, um für die Düse heiße Luft bereitzustellen.

8. Vorrichtung nach Anspruch 7, **dadurch gekennzeichnet, dass** eine Eindüseinrichtung zum Eindüsen von H₂O₂ in den Weg der Luft vorgesehen ist, wobei die Eindüseinrichtung stromauf oder stromab des Lufterhitzers vorgesehen sein kann.

9. Vorrichtung nach einem der Ansprüche 1 bis 8, **dadurch gekennzeichnet, dass** der Sterilisator (9) einen Sterilisationstunnel (27) zur Sterilisation der Flaschen (7) durch H₂O₂ von außen umfasst, wobei vorzugsweise H₂O₂ aus den Flaschen (7) in den Sterilisationstunnel (27) übertreten kann.

10. Vorrichtung nach Anspruch 9, **dadurch gekennzeichnet, dass** Zuleitungen von H₂O₂ und/oder heißem und/oder kaltem H₂O₂-Luft-Gemisch und/oder heißer und/oder kalter Luft zu dem Sterilisationstunnel (27) vorgesehen sind.

11. Vorrichtung nach einem der Ansprüche 1 bis 10, **dadurch gekennzeichnet, dass** der Flaschenweg unmittelbar stromauf des Sterilisators (9) von einem Warmhaltetunnel (8, 26) umgeben ist, in dem die Flaschen (7) auf der gewünschten Temperatur gehalten werden können, wobei vorzugsweise aus dem Sterilisator (9) warme, H₂O₂-haltige Luft in den Warmhaltetunnel (8, 26) übertreten kann.

12. Vorrichtung nach Anspruch 11, **dadurch gekennzeichnet, dass** am Übergang zwischen Warmhaltetunnel (8, 26) und Sterilisator (9) Zuleitungen zum Warmhaltetunnel (8, 26) für H₂O₂ und/oder heißes und/oder kaltes H₂O₂ -Luft-Gemisch vorgesehen sind.

13. Vorrichtung nach einem der Ansprüche 1 bis 12, **dadurch gekennzeichnet, dass** eine Düse oder ein Gasauslass vorgesehen ist, mit dem ein Sterilgas, wie z.B. Sterilluft, zum Ausblasen des H₂O₂ in die Flasche (7) eingeleitet werden kann, wobei eine Vorwärmeinrichtung zum Vorwärmen des Sterilgases vorgesehen ist.

14. Vorrichtung nach einem der Ansprüche 1 bis 13, **dadurch gekennzeichnet, dass** eine Vorwärmeinrichtung für Flaschenverschlüsse (15) vorgesehen ist, mit der Flaschenverschlüsse (15) durch Infrarotbestrahlung oder durch Heißluft erwärmt werden und zwar auf eine Temperatur oberhalb des Taupunkts von H₂O₂.

15. Vorrichtung nach Anspruch 14, **dadurch gekennzeichnet, dass** eine Beaufschlagungseinrichtung vorgesehen ist, mit der die Flaschenverschlüsse (15) mit H₂O₂ beaufschlagt werden können, so dass das H₂O₂ nicht kondensiert.

16. Vorrichtung nach einem der Ansprüche 14 oder 15, **dadurch gekennzeichnet, dass** ein Speicher für Flaschenverschlüsse vorgesehen ist, in dem die Flaschenverschlüsse (15) auf einer solchen Temperatur gehalten werden, so dass in dem Speicher vorhandenes H₂O₂ nicht kondensiert.

17. Vorrichtung nach einem der Ansprüche 1 bis 16, **dadurch gekennzeichnet, dass** die Mittel eine Ummantelung und/oder eine thermische Isolation und/oder Beheizung des Sterilisators (9) umfassen.

18. Vorrichtung nach einem der Ansprüche 1 bis 17, **dadurch gekennzeichnet, dass** die Mittel eine Ummantelung (22) und/oder thermische Isolation und/oder Beheizung von weiteren Flaschen- /Verschlussbehandlungsgeräten (17) umfassen, wobei die Wärme der Heizung aus einer Blasmaschine (4) und/oder einer dazugehörigen Heizstation (3) für Vorformlinge (2) gewonnen wird.

19. Verfahren zum Sterilabfüllen von Flüssigkeiten in Flaschen mit den Schritten:
- Sterilisieren der Flaschen mit H₂O₂ und
- Befüllen sowie Verschließen der Flaschen, wobei die Flaschen (7) beim Sterilisierung eine solche Temperatur haben, dass eine Kondensation des H₂O₂ auf der Flaschenoberfläche verhindert wird, wobei die Flaschen (7) bei einem Formblasvorgang diese Temperatur erhalten, **dadurch gekennzeichnet, dass** die Flaschen (7) nach dem Formblasvorgang durch Heißluft von innen auf die benötigte Temperatur gebracht werden.

20. Verfahren nach Anspruch 19, **dadurch gekennzeichnet, dass** die Flaschen (7) nach dem Formblasvorgang thermisch isoliert gefördert werden, um so einen übermäßigen Wärmeverlust der Flaschen (7) zu vermeiden.

21. Verfahren nach Anspruch 19, **dadurch gekennzeichnet, dass** die Flaschen (7) durch Infrarotbestrahlung on außen auf die benötigte Temperatur gebracht werden.

22. Verfahren nach Anspruch 21, **dadurch gekennzeichnet, dass** die Luft mit Dampf erhitzt wird.

23. Verfahren nach Anspruch 21 oder 22, **dadurch gekennzeichnet, dass** die Erwärmung der Flaschen bei Umlauf um ein Karussell (24) erfolgt, wobei die Flaschen (7) durch einen Tunnel (25) geführt werden.

24. Verfahren nach einem der Ansprüche 19 bis 23, **dadurch gekennzeichnet, dass** das H₂O₂ als reines Gas und/oder als Gas-Luftgemisch und/oder als Gas-Luft-Dampfgemisch in die Flaschen (7) eingeblasen wird und anschließend mit Luft wieder ausgeblasen wird.

25. Verfahren nach Anspruch 24, **dadurch gekennzeichnet, dass** zum Einblasen des H₂O₂ eine Düse oder ein Gasauslass in die Flasche (7) eingetaucht wird.

26. Verfahren nach Anspruch 24 oder 25, **dadurch gekennzeichnet, dass** das H₂O₂ in einen Luftweg eingedüst wird, wobei die Eindüsung stromauf oder stromab eines Lufterhitzers erfolgen kann.

27. Verfahren nach einem der Ansprüche 19 bis 26, **dadurch gekennzeichnet, dass** die Flaschen (7) von außen mit H₂O₂ zur Sterilisation in Kontakt gebracht werden, wobei sich die Flaschen (7) dabei in einem Sterilisationstunnel (27) befinden.

28. Verfahren nach Anspruch 27, **dadurch gekennzeichnet, dass** das H₂O₂ für den Außenkontakt aus dem Flascheninneren ausströmt und/oder separat den Flaschen (7) von außen in den Sterilisationstunnel (27) zugeführt wird.

29. Verfahren nach einem der Ansprüche 19 bis 28, **dadurch gekennzeichnet, dass** die Flaschen (7) nach dem Erwärmen zu dem Sterilisator in einem Warmhaltetunnel (8, 26) mit einer warmen und H₂O₂ -haltigen Atmosphäre transportiert werden, um die Flaschen (7) auf der benötigten Temperatur zu halten.

30. Verfahren nach Anspruch 29, **dadurch gekennzeichnet, dass** warme und H₂O₂-haltige Luft aus dem Sterilisator (9) in dem Warmhaltetunnel (8, 26) übertritt und/oder H₂O₂ und/oder heißes und/oder kaltes H₂O₂ -Luft-Gemisch und/oder heiße und/oder kalte Luft dem Warmhaltetunnel (8, 26) zugeführt wird.

31. Verfahren nach einem der Ansprüche 19 bis 30, **dadurch gekennzeichnet, dass** die Flaschenverschlüsse (15) vorgewärmt und gleichzeitig und/oder anschließend zur Sterilisation mit H₂O₂ beaufschlagt werden.

32. Verfahren nach Anspruch 31, **dadurch gekennzeichnet, dass** die Flaschenverschlüsse (15) in einem Speicher für Flaschenverschlüsse (15) mit H₂O₂ beaufschlagt werden.

33. Verfahren nach einem der Ansprüche 19 bis 32, **dadurch gekennzeichnet, dass** der Sterilisator (9) beheizt wird und/oder auf einer erhöhten Temperatur gehalten wird, so dass eine Kondensation von H₂O₂ auf Teilen des Sterilisators (9) verhindert wird.

34. Verfahren nach einem der Ansprüche 19 bis 33, **gekennzeichnet durch** eine Speicherung der Flaschen (7) zwischen Formblasen und Sterilisieren im warmen Zustand.

35. Verfahren nach einem der Ansprüche 19 bis 34, **dadurch gekennzeichnet, dass** die Flaschen (7) bei der Sterilisation eine Temperatur zwischen 50° bis 70° Celsius, vorzugsweise zwischen 50° bis 60° Celsius haben.

36. Verfahren nach einem der Ansprüche 19 bis 35, **dadurch gekennzeichnet, dass** die verwendete Luft ionisiert wird.

## Claims

1. Device for the sterile bottling of liquids, comprising
- a steriliser for sterilising the bottles with H₂O₂,
- a filler for filling the bottles and
- a sealer for applying a closure, such as, e.g. a cap, means for adjusting the temperature of the bottles (7) being provided in order to prevent the condensation of H₂O₂ on the surface of the bottles and the means including coolants provided in a blow-moulding machine (4) for producing the bottles in order to adjust the temperature of the bottles (7) when they are discharged from the blow-moulding machine (4), **characterised in that** the means further include a heating device (23) for heating the path of the bottles (7) to the steriliser (9), the heating device (23) including an internal heating device for blowing preheated air into the bottles (7).

2. Device according to claim 1, **characterised in that** the means include sheathing and/or thermal insulation of the path (6) of the bottles (7) between the blow-moulding machine (4) and the steriliser (9).

3. Device according to claim 1, **characterised by** a supply of steam for preheating the air in order to bring the air to a temperature above the desired temperature for the bottles (7).

4. Device according to claim 1, **characterised in that** the heating device (23) includes an external heating device for heating the bottles (7) from the outside, the external heating device including a hot-air blower for blowing air against the bottles (7) from the outside.

5. Device according to claim 1, **characterised in that** the heating device (23) includes a tunnel (25) around the bottle path.

6. Device according to one of claims 1 to 5, **characterised by** at least one nozzle or at least one gas outlet for introducing H₂O₂ in gaseous form into the bottles (7), the nozzle or gas outlet preferably being positioned in such a manner that it can be immersed in the bottle.

7. Device according to claim 6, **characterised in that** a heat exchanger and/or an electric heater is provided as an air heater in order to provide hot air for the nozzle.

8. Device according to claim 7, **characterised by** an injection device for injecting H₂O₂ into the path of the air, wherein the injection device can be provided upstream or downstream of the air heater.

9. Device according to one of claims 1 to 8, **characterised in that** the steriliser (9) includes a sterilising tunnel (27) for sterilising the bottles (7) from the outside with H₂O₂, wherein H₂O₂ can preferably flow out of the bottles (7) into the sterilising tunnel (27).

10. Device according to claim 9, **characterised by** lines for supplying H₂O₂ and/or a hot and/or cold H₂O₂-air mixture and/or hot and/or cold air to the sterilising tunnel (27).

11. Device according to one of claims 1 to 10, **characterised in that** the bottle path is surrounded immediately upstream of the steriliser (9) by a holding tunnel (8, 26) in which the bottles (7) can be kept at the desired temperature, wherein warm, H₂O₂-containing air can preferably flow out of the steriliser (9) into the holding tunnel (8, 26).

12. Device according to claim 11, **characterised in that** lines for supplying H₂O₂ and/or a hot and/or cold H₂O₂-air mixture to the holding tunnel (8, 26) are provided at the transition between the holding tunnel (8, 26) and the steriliser (9).

13. Device according to one of claims 1 to 12, **characterised by** a nozzle or a gas outlet for introducing a sterile gas, such as, e.g. sterile air, into the bottle (7) in order to blow out the H₂O₂, a preheating device being provided in order to preheat the sterile gas.

14. Device according to one of claims 1 to 13, **characterised by** a preheating device for bottle closures (15), for heating bottle closures (15) to a temperature above the dew point of H₂O₂ by infrared irradiation or by hot air.

15. Device according to claim 14, **characterised by** a device for supplying H₂O₂ to the bottle closures (15) in such a manner that the H₂O₂ does not condense.

16. Device according to either of claims 14 or 15, **characterised by** a store for bottle closures in which the bottle closures (15) are kept at a temperature such that H₂O₂ present in the store does not condense.

17. Device according to one of claims 1 to 16, **characterised in that** the means include sheathing and/or thermal insulation and/or heating of the steriliser (9).

18. Device according to one of claims 1 to 17, **characterised in that** the means include the sheathing (22) and/or thermal insulation and/or heating of further equipment (17) for treating bottles/closures, the heat for the heating being obtained from a blow-moulding machine (4) and/or an associated heating station (3) for parisons (2).

19. Method for the sterile bottling of liquids, comprising the steps:
- sterilising the bottles with H₂O₂,
- filling and sealing the bottles, the temperature of the bottles (7) during the sterilisation process being such that the condensation of H₂O₂ on the surface of the bottles is prevented and this temperature being imparted to the bottles (7) during a blow-moulding process, **characterised in that** the bottles (7) are brought to the required temperature from the inside by hot air after the blow-moulding process.

20. Method according to claim 19, **characterised in that** the bottles (7) are conveyed in a thermally insulated manner after the blow-moulding process in order to prevent excessive heat loss from the bottles (7).

21. Method according to claim 19, **characterised in that** the bottles (7) are brought to the required temperature from the outside by infrared irradiation.

22. Method according to claim 21, **characterised in that** the air is heated with steam.

23. Method according to claim 21 or claim 22, **characterised in that** the bottles are heated as they circulate around a carousel (24), the bottles (7) being guided through a tunnel (25).

24. Method according to one of claims 19 to 23, **characterised in that** the H₂O₂ is blown into the bottles (7) as a pure gas and/or as a gas-air mixture and/or as a gas-air-steam mixture and is then blown out again with air.

25. Method according to claim 24, **characterised in that** a nozzle or a gas outlet is immersed in the bottle (7) in order to blow in the H₂O₂.

26. Method according to claim 24 or claim 25, **characterised in that** the H₂O₂ is injected into an air path, wherein the injection can be effected upstream or downstream of an air heater.

27. Method according to one of claims 19 to 26, **characterised in that** the bottles (7) are brought into contact from the outside with H₂O₂ for sterilisation, the bottles (7) being situated in a sterilising tunnel (27).

28. Method according to claim 27, **characterised in that** the H₂O₂ for the external contact flows out of the interior of the bottles and/or is supplied separately from the outside to the bottles (7) in the sterilising tunnel (27).

29. Method according to one of claims 19 to 28, **characterised in that**, after heating, the bottles (7) are transported to the steriliser in a holding tunnel (8, 26) with a warm, H₂O₂-containing atmosphere in order to keep the bottles (7) at the required temperature.

30. Method according to claim 29, **characterised in that** warm, H₂O₂-containing air flows out of the steriliser (9) into the holding tunnel (8, 26) and/or H₂O₂ and/or a hot and/or cold H₂O₂-air mixture and/or hot and/or cold air is supplied to the holding tunnel (8, 26).

31. Method according to one of claims 19 to 30, **characterised in that** the bottle closures (15) are preheated and H₂O₂ is supplied simultaneously and/or subsequently thereto for sterilisation.

32. Method according to claim 31, **characterised in that** H₂O₂ is supplied to the bottle closures (15) in a store for bottle closures (15).

33. Method according to one of claims 19 to 32, **characterised in that** the steriliser (9) is heated and/or kept at an elevated temperature in order to prevent the condensation of H₂O₂ on parts of the steriliser (9).

34. Method according to one of claims 19 to 33, **characterised in that** the bottles (7) are stored in a warm state between the blow moulding and sterilisation processes.

35. Method according to one of claims 19 to 34, **characterised in that** the bottles (7) have a temperature of between 50°C and 70°C, preferably between 50°C and 60°C, during the sterilisation process.

36. Method according to one of claims 19 to 35, **characterised in that** the air used is ionised.

## Revendications

1. Dispositif de remplissage stérile de bouteilles avec des liquides comprenant :
- un élément de stérilisation pour stériliser les bouteilles avec H₂O₂,
- un élément de remplissage pour remplir les bouteilles, et
- un élément de fermeture pour la mise en place d'une obturation par exemple d'un capuchon d'obturation, des moyens étant prévus pour permettre de régler la température des bouteilles (7) de façon à éviter une condensation de l'H₂O₂ sur leur surface, ces moyens renfermant des moyens de refroidissement montés dans une machine de formage par soufflage (4) avec laquelle les bouteilles sont fabriquées, et permettant de régler la température des bouteilles (7) lors de leur sortie de la machine de formage (4),
**caractérisé en ce que**
les moyens comportent en outre un dispositif de chauffage (23) de la trajectoire des bouteilles (7) vers l'élément de stérilisation (9), ce dispositif de chauffage (23) comprenant un dispositif de chauffage interne par lequel de l'air préchauffé peut être soufflé dans les bouteilles (7).

2. Dispositif conforme à la revendication 1,
**caractérisé en ce que**
les moyens comportent une enveloppe et/ou une isolation thermique de la trajectoire (6) des bouteilles (7) entre la machine de soufflage (4) et l'élément de stérilisation (9).

3. Dispositif conforme à la revendication 1,
**caractérisé en ce que**
pour préchauffer l'air, il est prévu une arrivée de vapeur permettant l'amenée d'air à une température située au-dessus de la température des bouteilles (7) souhaitée.

4. Dispositif conforme à la revendication 1,
**caractérisé en ce que**
le dispositif de chauffage (23) comporte un dispositif de chauffage externe permettant de chauffer les bouteilles (7) de l'extérieur, ce dispositif de chauffage externe comprenant un organe de soufflage d'air chaud sur les bouteilles (7) à partir de l'extérieur.

5. Dispositif conforme à la revendication 1,
**caractérisé en ce que**
le dispositif de chauffage (23) comprend un tunnel (25) situé autour de la trajectoire des bouteilles.

6. Dispositif conforme à l'une des revendications 1 à 5,
**caractérisé en ce qu'**
il comporte au moins une buse ou au moins une sortie de gaz pour amener le H₂O₂ sous forme gazeuse dans les bouteilles (7), cette buse ou cette sortie de gaz étant de préférence montée de façon à pouvoir être plongée dans les bouteilles.

7. Dispositif conforme à la revendication 6,
**caractérisé en ce qu'**
en tant qu'élément de chauffage d'air il est prévu un échangeur de chaleur et/ou un élément de chauffage électrique pour fournir de l'air chaud à la buse.

8. Dispositif conforme à la revendication 7,
**caractérisé en ce qu'**
il comporte un dispositif de projection pour projeter du H₂O₂ dans la trajectoire de l'air, ce dispositif de projection pouvant être monté en amont ou en aval de l'élément de chauffage de l'air.

9. Dispositif conforme à l'une des revendications 1 à 8,
**caractérisé en ce que**
l'élément de stérilisation (9) comporte un tunnel de stérilisation (27) pour permettre la stérilisation des bouteilles (7) par H₂O₂ à partir de l'extérieur, le H₂O₂ pouvant de préférence passer des bouteilles (7) dans le tunnel de stérilisation (27).

10. Dispositif conforme à la revendication 9,
**caractérisé en ce qu'**
il comporte des conduites de transfert de H₂O₂ et/ou d'un mélange H₂O₂-air chaud et/ou froid et/ou d'air chaud et/ou froid vers le tunnel de stérilisation (27).

11. Dispositif conforme à l'une des revendications 1 à 10,
**caractérisé en ce que**
la trajectoire des bouteilles est entourée, directement en amont de l'élément de stérilisation (9) par un tunnel de maintien de la chaleur (8, 26) dans lequel les bouteilles (7) peuvent être maintenues à la température souhaitée, de l'air chaud renfermant du H₂O₂ pouvant de préférence passer de l'élément de stérilisation (9) dans le tunnel de maintien de la chaleur (8, 26).

12. Dispositif conforme à la revendication 11,
**caractérisé en ce qu'**
au niveau de la transition entre le tunnel de maintien de la chaleur (8, 26) et l'élément de stérilisation (9) il est prévu des conduites de transfert vers le tunnel de maintien de la chaleur (8, 26) pour H₂O₂ et/ou un mélange H₂O₂-air chaud et/ ou froid.

13. Dispositif conforme à l'une des revendications 1 à 12,
**caractérisé en ce qu'**
il comporte une buse ou une sortie de gaz permettant l'introduction d'un gaz stérile tel que par exemple de l'air stérile pour souffler du H₂O₂ dans les bouteilles (7), un dispositif de préchauffage étant prévu pour préchauffer ce gaz stérile.

14. Dispositif conforme à l'une des revendications 1 à 13,
**caractérisé en ce qu'**
il comporte un dispositif de préchauffage des éléments de fermeture des bouteilles (15) par lequel ces éléments de fermeture (15) peuvent être chauffés par rayonnement infrarouge ou par de l'air chaud, ce à une température située au-dessus du point de rosée de H₂O₂.

15. Dispositif conforme à la revendication 14,
**caractérisé en ce qu'**
il comporte un dispositif d'alimentation permettant d'alimenter les éléments de fermeture des bouteilles (15) en H₂O₂ de sorte que l'H₂O₂ ne se condense pas.

16. Dispositif conforme à l'une des revendications 14 ou 15,
**caractérisé en ce qu'**
il comporte un accumulateur pour les éléments de fermeture des bouteilles dans lequel ces éléments de fermeture des bouteilles (15) sont maintenus à une température telle que l'H₂O₂ présent dans cet accumulateur ne se condense pas.

17. Dispositif conforme à l'une des revendications 1 à 16,
**caractérisé en ce que**
les moyens renferment une enveloppe et/ou une isolation thermique et/ou un organe de chauffage de l'élément de stérilisation (9).

18. Dispositif conforme à l'une des revendications 1 à 17,
**caractérisé en ce que**
les moyens comportent une enveloppe (22) et/ou une isolation thermique et/ou un organe de chauffage d'autres appareils de traitement des bouteilles de fermeture (17), la chaleur de chauffage étant obtenue à partir d'une machine de soufflage (4) et/ou d'un poste de soufflage (3) associé à celle-ci pour des ébauches préformées (2).

19. Procédé de remplissage stérile de bouteilles avec des liquides comprenant les étapes consistant à :
- stériliser les bouteilles avec H₂O₂, et
- remplir et fermer les bouteilles, les bouteilles (7) ayant lors de la stérilisation une température telle qu'une condensation de l'H₂O₂ sur leur surface soit évitée, les bouteilles (7) acquérant cette température lors d'un procédé de formage par soufflage,
**caractérisé en ce qu'**
après le procédé de formage par soufflage, les bouteilles (7) sont amenées à la température nécessaire par de l'air chaud, à partir de l'extérieur.

20. Procédé conforme à la revendication 19,
**caractérisé en ce qu'**
après le procédé de formage par soufflage, les bouteilles (7) sont fournies en étant thermiquement isolées de façon de permettre d'éviter ainsi une perte de chaleur excessive de ces bouteilles (7).

21. Procédé conforme à la revendication 19,
**caractérisé en ce que**
les bouteilles (7) sont amenées à la température nécessaire par rayonnement infrarouge à partir de l'extérieur.

22. Procédé conforme à la revendication 21,
**caractérisé en ce que**
l'air est chauffé avec de la vapeur.

23. Procédé conforme à la revendication 21 ou 22,
**caractérisé en ce que**
le chauffage des bouteilles est effectué au cours de leur rotation autour d'un carrousel (24), ces bouteilles (7) étant transférées au travers d'un tunnel (25).

24. Procédé conforme à l'une des revendications 19 à 23,
**caractérisé en ce que**
le H₂O₂ est soufflé dans les bouteilles (7) sous la forme de gaz pur et/ou sous la forme d'un mélange gaz-air et/ou sous la forme d'un mélange vapeur-gaz-air, puis, de l'air est à nouveau soufflé.

25. Procédé conforme à la revendication 24,
**caractérisé en ce que**
pour souffler le H₂O₂, on plonge une buse ou une sortie de gaz dans les bouteilles (7).

26. Procédé conforme à la revendication 24 ou 25,
**caractérisé en ce que**
le H₂O₂ est soufflé dans une trajectoire d'air, ce soufflage pouvant être effectué en amont ou en aval d'un élément de chauffage de l'air.

27. Procédé conforme à l'une des revendications 19 à 26,
**caractérisé en ce que**
les bouteilles (7) sont mises en contact par l'extérieur avec le H₂O₂ pour permettre leur stérilisation, ces bouteilles (7) se trouvant alors dans un tunnel de stérilisation (27).

28. Procédé conforme à la revendication 27,
**caractérisé en ce que**
le H₂O₂ pour le contact externe sort de l'intérieur des bouteilles et/ou est transféré séparément des bouteilles (7) par l'extérieur dans le tunnel de stérilisation (27).

29. Procédé conforme à l'une des revendications 19 à 28,
**caractérisé en ce qu'**
après avoir été chauffées, les bouteilles (7) sont transportées vers l'élément de stérilisation dans un tunnel de maintien de la chaleur (8, 26) avec une atmosphère chaude renfermant du H₂O₂ pour maintenir les bouteilles (7) à la température nécessaire.

30. Procédé conforme à la revendication 29,
**caractérisé en ce que**
de l'air chaud renfermant de l'H₂O₂ sortant du stérilisateur (9) passe dans le tunnel de maintien de la chaleur (8, 26) et/ou du H₂O₂ et/ou un mélange H₂O₂-air chaud et/ou froid et/ou de l'air chaud et/ou froid est transféré dans le tunnel de maintien de la chaleur (8, 26).

31. Procédé conforme à l'une des revendications 19 à 30,
**caractérisé en ce que**
les éléments de fermeture des bouteilles (15) sont préchauffés et sont simultanément et/ ou ensuite alimentés pour permettre leur stérilisation par H₂O₂.

32. Procédé conforme à la revendication 31,
**caractérisé en ce que**
les éléments de fermeture des bouteilles (15) sont alimentés avec le H₂O₂ dans un accumulateur de tels éléments de fermeture des bouteilles (15).

33. Procédé conforme à l'une des revendications 19 à 32,
**caractérisé en ce que**
l'élément de stérilisation (9) est chauffé et/ou maintenu à une température élevée de façon à éviter une condensation de H₂O₂ sur des parties de cet élément de stérilisation (9).

34. Procédé conforme à l'une des revendications 19 à 33,
**caractérisé par**
une accumulation des bouteilles (7) entre leur formage par soufflage et leur stérilisation à l'état chaud.

35. Procédé conforme à l'une des revendications 19 à 34,
**caractérisé en ce que**
lors de leur stérilisation les bouteilles (7) ont une température comprise entre 50 et 70°C, de préférence entre 50 et 60°C.

36. Procédé conforme à l'une des revendications 19 à 35,
**caractérisé en ce que**
l'air utilisé est ionisé.
